# EUROPEAN PATENT APPLICATION

(11) **EP 3 718 505 A1**
(43) Date of publication of application: **07.10.2020**
(21) Application number: 19167599.0
(22) Date of filing: 05.04.2019
(51) Int. Cl.: A61F 2/01, A61F 2/95

(54) **TRANSCATHETER ANTI EMBOLIC FILTER FOR ARTERIAL AND VENOUS VESSELS**

(71) Applicant: AORTICLAB SARL, 1073 Savigny (CH)
(72) Inventor: BONETTI, Francesco, 10121 Torino (IT); OSTA, Stefano, 13040 Saluggia (IT); PASQUINO, Enrico, 1073 Savigny (CH); OSTA, Franco, 15020 Mombello Monferrato (AL) (IT); BAI, Yi Peng, 20159 Milano (IT); PARIGI, Giulia, 20133 Milano (IT)
(74) Representative: Grosfillier, Philippe

(57) **Abstract**

An intra-vessel transcatheter filter device (1) designed to capture and remove emboli, thus preventing distal embolization, comprising:
• a tubular filter (2) comprising a flexible porous material and defined by a distal element and a proximal element, namely a main body (3) and a funnel (4);
i. said main body (3) having a length adapted to extend within a suitable vessel zone; said main body (3) comprising:
a) a distal end (5) that is adapted to be radially coupled with said zone and hermetically sealed to it when the device is in an active configuration, said distal end (5) being provided of selectively actionable closure means so that said distal end is designed to be open when the device is in an active configuration and closed before the device retraction,
b) a proximal end (6);

ii. said funnel (4) forming an extension of said main body (3), with the funnel base located at said proximal end (6).

• a support structure assembly (8) comprising:
i. a supporting catheter (11) extending within said main body (3),
ii. one radially expandable distal structure (9) fixed to said distal end (5),
iii. one radially expandable proximal structure (10) positioned in correspondence of said proximal end (6),

said distal structure (9) and said proximal structure (10) being fixed at least in an end to said supporting catheter (11).

## Description

### FIELD OF INVENTION

The present invention generally relates to a transcatheter anti embolic filter, in particular to an intra-aortic filter to be used to protect cerebral and peripheral vessels from potential dissemination of emboli.

### BACKGROUND OF THE INVENTION

The clinical complications related to the implant of a transcatheter heart valve prosthesis (TAVI) are mainly related to the fact that it overlaps the diseased native valve. The heavy presence of tissue calcifications, involving the valve apparatus and the surrounding tissues, influences the correct deployment of the prosthesis creating the conditions for embolic episodes.

The procedural embolic events, so called "macro-embolic cerebral events", are occurring during a TAVI implant procedure (during predilation, implant or postdilation) and are mainly related to the embolization of macro debris of calcium of fibroelatic particles usually targeting the brain (strokes), the coronary arteries or the peripheral organs. However, the strokes are the most frightful clinical events occurring, nowadays, at a rate of 2.7% against a rate of 3.3% of the previous generations of TAVIs. This reduction of strokes is related to the minor need of pre- and postdilation during TAVI implant nevertheless this data are unclear since are referring to aortic valves with a mild level of calcification. The post-procedural micro-embolic cerebral events are documented in at least 8% of the patients submitted to investigation. The high incidence of new cerebral lesions after TAVI warrants for a longer term evaluation of neurocognitive function.

In this study conducted over a short-term follow-up period of 3 months, no impairment of neurocognitive function was observed clinically, and the majority of lesions (80%) had resolved on 3-months MRI. However, the issue of periprocedural brain embolization and its potential effects on neurocognitive function may portend greater clinical implications once the indication for TAVI is broadened to include younger patients with long life expectancy.

Future research in the field of TAVI should thus be directed at developing strategies to reduce the risk of embolization (e.g., less traumatic, smaller-bore catheter systems, improved identification of patients at risk for embolization and a potential use of cerebral protection devices).

In some clinical studies at least 10% of the patients, submitted to TAVI implant, show a neurological damage detectable during psychometric tests. While this occurrence rate can be acceptable in high risk and an old patient population it appears unacceptable in lower-risk younger patients. Several clinical studies are ongoing to better investigate this clinical condition.

Another kind of embolic events are the sub-acute and chronic microembolic events occurring after the immediate post-procedural time. The native aortic calcific valve is rough, with a warty surface, immobilized acting like an atherosclerotic ulcerated plaque. This condition is favouring the formation of microtrombi that later-on embolize towards the brain and other peripheral organs. The native aortic valve left in place as a source of microemboli has been taken into account in several clinical studies that demonstrated their role in the onset of vascular origin dementia. This evidence creates a concern when the TAVI are implanted in younger patients where an acceleration of the vascular dementia could impact in a serious way on the social costs.

In summary the periprocedural clinical complications following a TAVI implant are strongly related to the presence of the heavily calcified aortic valve left in place. It brings, acutely, an occurrence of macro-embolic cerebral events (strokes) and hemodynamic consequences such as the PVLs resulting in a various severity of aortic valve insufficiency. These unsatisfactory clinical outcomes are closely related to an irregular deployment of the transcatheter valve prostheses in concomitance of highly calcified aortic native valves. The longer term clinical complications are characterized by the cerebral micro-embolizations generated by the native aortic valve leaflets' left in place that become a source of emboli responsible for vascular dementia.

The overall rate of clinical complications in TAVI is ranging between 5% and 12%. This occurrence is most probably underestimated because it does not include patients with highly calcified and biscuspid native valves.

These evidences highlight the importance of protecting the peripheral organs, in particular the brain and the heart, against embolizations occurring during TAVIs procedures.

The increasing overall use of TAVI respect to SAVR, and the higher rate of intermediate risk patients implanted with TAVI, both are indicating the convenience of adopting embolic protection to optimize the long-term survival and quality of life of these patients.

AKI (Acute Kidney Injury) is a frequent complication after TAVI being reported in ranges from 8.3% to 58%. Differing results might partially be explained by the use of different definitions of AKI. In general, this complication is correlated to comorbidities, access route (transfemoral, transapical or others) and amount of contrast liquid used during the procedure. There aren't clinical study on the correlation between the embolization process that occurs during the TAVI procedure and AKI, due also to the absence of a device that can capture the emboli direct to the renal zone, but it's possible to think that the cloud of embolization detached from the valve during the procedure can help the occurrence of this complication.

The abovementioned complications associated to TAVI procedures apply also to other transcatheter procedures, such as valvuloplasty (when unassociated to TAVI), native valve repair and heart recovery procedures, all conditions potentially leading to emboli release from ventricle, native valve or thoracic aorta. Furthermore, catheter navigation itself along a calcified aorta, can make calcification dislodgement and emboli release.

Furthermore, emboli complications are shown in transcatheter procedures other than intra-aortic ones, therefore an antiembolic protection can be highly recommended also for other districts.

Actually, Patents Application including emboli protection were filed since 20 years ago, such as the invention disclosed in US 6,361,545, disclosing a perfusion filter catheter able to be adopted in the frame of SAVR and cardiopulmonary bypass procedures and AU 2011202667, disclosing and embolic filter apparatus and method for heart valve replacement.

Nowadays, there are only few devices in clinical use that protect cerebral and peripheral circulation on the frame of transcatheter cardiac and aortic procedures.

The deflector devices, deflect emboli from the brachiocephalic trunk and the left common carotid artery towards the peripheral circulation: therefore, they only impede debris entering in the cerebral vessels and diverting them to the peripheral circulation. Moreover, in case of dislodgement from their intended position, the diverting function is missed.

The antiembolic filter on the market, whose main characteristics are disclosed in United States Patent Application Publication US 2018177582, actually captures emboli with a mesh, but only cover two of the three cerebral vessels and not the peripheral circulation.

Other Patents, as United States Patent Application Publication US 2014/0005540 and US 2016/0235515, disclose an embolic protection device filter which is able to protect the cerebral and systemic circulation, although they show some difficulties on the interaction with other working catheters, such as the ones bearing TAVI devices, that need to be positioned before the filter deployments; moreover, during the TAVI positioning, being its catheter outside the filter protection, a small area of the aorta results unprotected; finally, in case TAVI repositioning in descending aorta is required, it would be needed to temporary remove the filter protection.

United States Patent Application Publication US 2018/0110607, discloses an embolic protection device filter which is able to protect the cerebral and systemic circulation; the device has a collection chamber for emboli captured containment, and allows the passage of other catheters inside its cylindrical body. Some disadvantages are shown by the mesh pore size, whose range is defined in the range of about 1mm to about 0,1 mm, and by the absence of a distal closure mechanism that inherently would prevent upstream release of emboli at closure.

International patent application WO 2017/042808 discloses an embolic protection device including a distal porous deflector covering cerebral vessels connected to a proximal emboli collector comprising at least one filter pocket able to be crossed. Although the distal deflector can appear advantageous in terms of encumbrance respect to a full filter configuration, it actually shows disadvantages when interacting with other working catheters, which can result in a loss of cerebral protection in case of small deflector movements.

International patent application WO 2015/185870 and WO 2018/211344 both disclose a filter device, including a temporary valve prosthesis, designed to be inserted in aorta. Both devices provide improvements with respect to other prior art devices. They however show some drawbacks, such as their positioning proximal respect to the native valve, that limits the possibility to directly operate onto it, and the difficulty to insert additional devices through the prosthesis due to catheter dimensional constraints.

### SUMMARY OF THE INVENTION

The occurrence of clinical events, as discussed in the previous chapter, are prevented with the present invention, which consists of an antiembolic filter. The filter has a proximal funnel that allows working catheters crossing a generally closed filter port, whilst preventing downstream collected emboli release; this allows the working catheters of accessories and / or transcatheter devices be tracked inside the filter without directly contacting the vessel after, contributing to prevent vessel wall injuries and relevant calcification detachment, whilst preventing emboli release. In addition, the filter has a distal closure mechanism, to be used prior to retrieve the device preventing upstream emboli release at closure. Furthermore, protection of cerebral and peripheral circulation is guaranteed both for macroemboli and microemboli, thanks to adequate filter mesh pore selection.

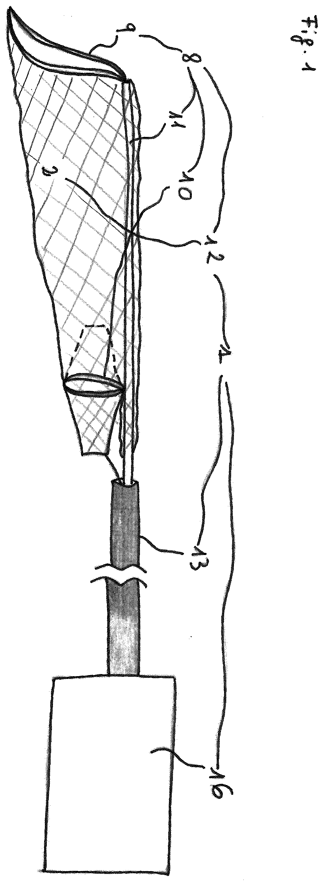

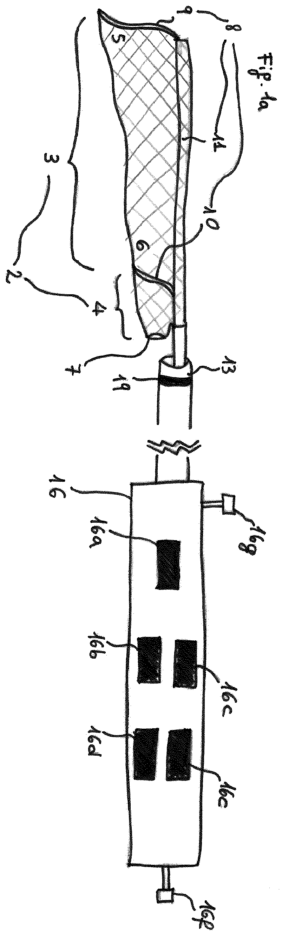

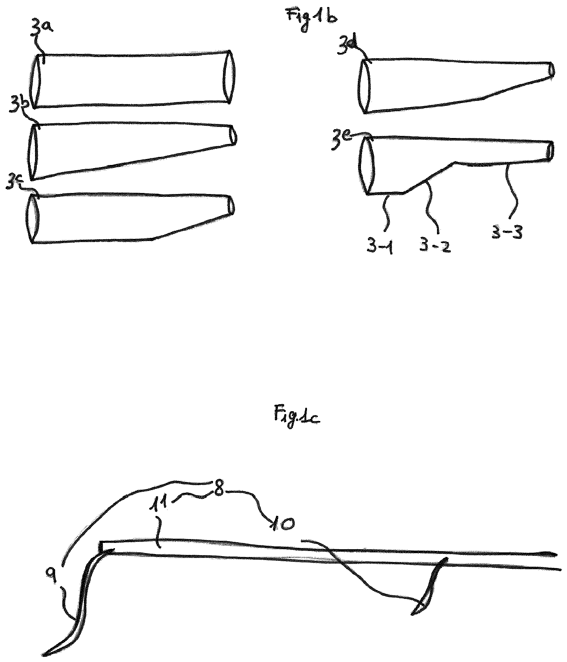

A transcatheter intraprocedural filter prosthesis (1) for blood vessel (in particular aorta vessel) comprising expandable (self-expandable) distal (9) and proximal (10) support structures, a tubular filter (2), an internal catheter (11), an external shaft (13), a handle (16) (Fig. 1, 1a); said tubular filter (2) forming a tubular shape when deployed, with a distal end being normally open and a proximal port (7) normally closed. The complete collapsing and deployment of the filter is enabled by the relative linear movement of the external shaft (13) respect to the internal supporting catheter (11).

The functional element (tubular filter 2 and supports 9, 10) and the delivery system (catheters 11 and 13, handle 16) can be permanently joined.

In a specific intra-aortic embodiment, the distal end of the deployed filter (5) is positioned in ascending aorta (23), upstream respect to innominate artery (24), and the proximal end (6) is positioned in descending aorta (26), downstream respect to the end of aortic arch (25).

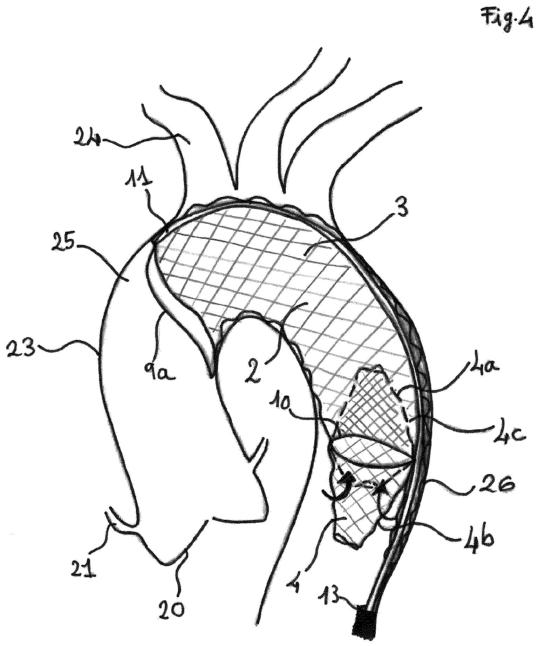

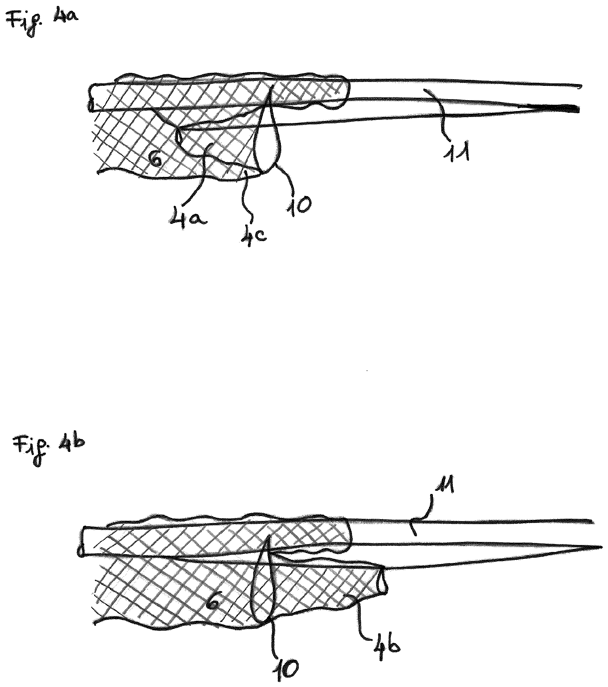

In another specific embodiment, the funnel (4) configuration can be modified during the procedure by maintaining its apex downstream (Fig. 4b) or reverted inside the filter main body (3: Fig. 4a) or in an intermediate position.

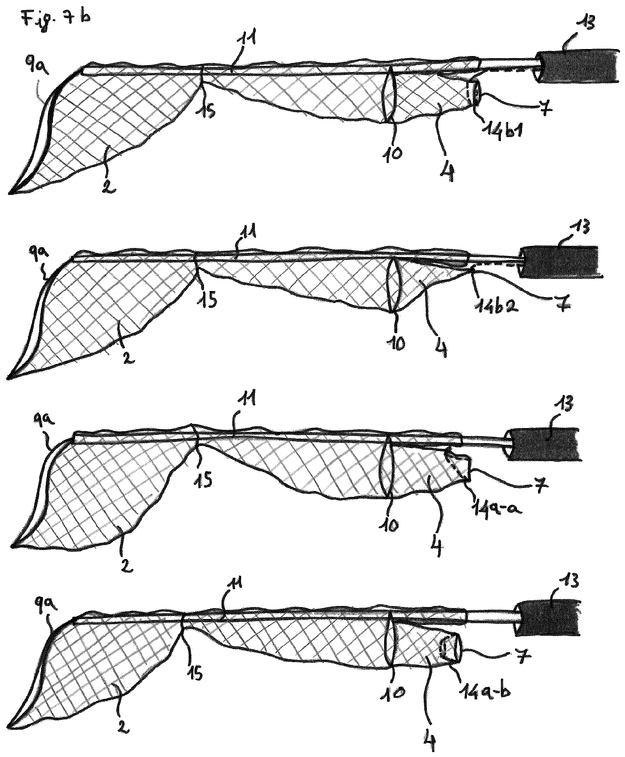

The device can be completely or partially collapsed during the procedure in order to be re-positioned. At the end of the procedure both the distal and proximal closure mechanisms are activated (Fig. 7b), then the device is collapsed, retracted inside the shaft and fully retrieved out from the patient.

The filter device (1) is intended to be inserted prior to start other transcatheter procedures and to be retrieved after other transcatheter devices removal.

In summary, the filter device here described is adapted to guarantee an antiembolic protection ensuring navigation of other working catheters into the filter, permanent closure at the proximal end and closure at the distal end before filter retrieve, thus giving advantages respect to existing devices and methods.

### DETAILED DESCRIPTION OF THE INVENTION

The invention will be better understood below, in association with some illustrated examples.

### Brief Description of the figures

- **Fig.1**: **Filter device assembly** in the active configuration, including a filter-structure-catheter assembly (12), an external shaft (13), a handle (16)
- **Fig. 1a**: **Filter device components nomenclature**
- **Fig. 1b**: **Main body of the filter** (3): some examples of geometries (a) cylindrical shape, b) conical shape, c) cylindro-conical shape, d) double conical shape, e) multi angle shape)
- **Fig. 1c**: **Support structure assembly** example (8)
- **Fig. 2**: **Two distal rings structure embodiment** (9b) example in the active configuration
- **Fig. 2a**: Adaptative mechanism of the two distal rings structure embodiment
- **Fig. 2b**: Retraction mechanism of the two distal rings structure embodiment
- **Fig. 3**: **One distal ring structure embodiment** (9a) example in the active configuration
- **Fig. 3a**: Adaptative mechanism of the one distal ring structure embodiment
- **Fig. 3b**: Retraction mechanism of the one distal ring structure embodiment
- **Fig. 4**: **Movable proximal funnel** embodiment (4)
- **Fig. 4a**: Movable funnel in the not active configuration (funnel downstream)
- **Fig. 4b**: Movable funnel in the active configuration (funnel reverted inside main body)
- **Fig. 5**: **Fixed funnel embodiment** (4) example, with a "8" shaped proximal structure (10)
- **Fig. 6**: **Fixed funnel embodiment** (4), with a ring shaped proximal structure (10)
- **Fig. 7a**: **Distal closure mechanism** (15) example
- **Fig. 7b**: **Proximal closure mechanism** (14) example
- **Fig. 8**: **Rings configuration** examples
- **Fig. 9**: **Integration of the movement mechanisms** for distal structure (9b) and funnel (4)
- **Fig. 10**: **Trackability and Navigation tools** example: external shaft (13), radiopaque marker (19) and tip (17)
- **Fig. 11a-g**: **Method for embolic protection: a intra-aortic procedure example**

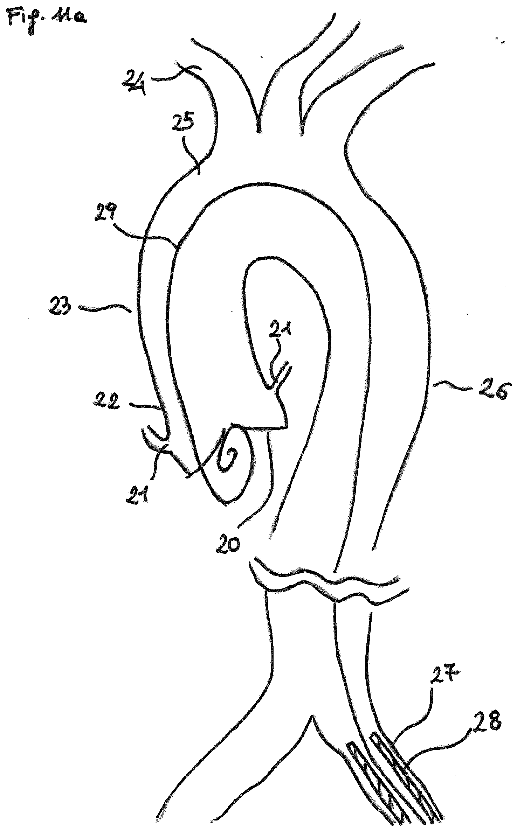

- **Fig. 11a**: Ascending aorta with **guide wire** (29)

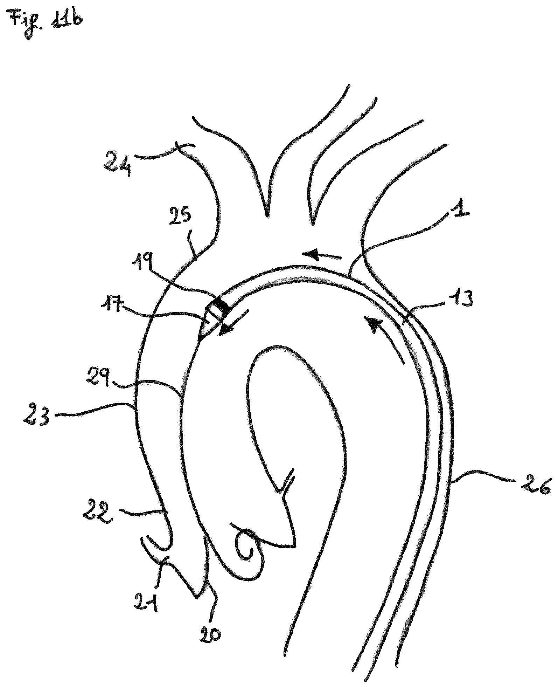

- **Fig. 11b**: **Navigation of** the collapsed filter device (1) along the aortic arch (25)

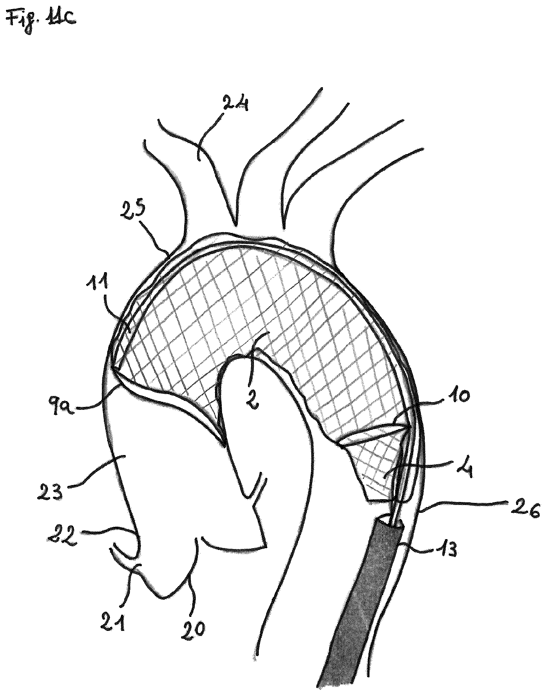

- **Fig. 11c**: **Device deployment** at the intended location

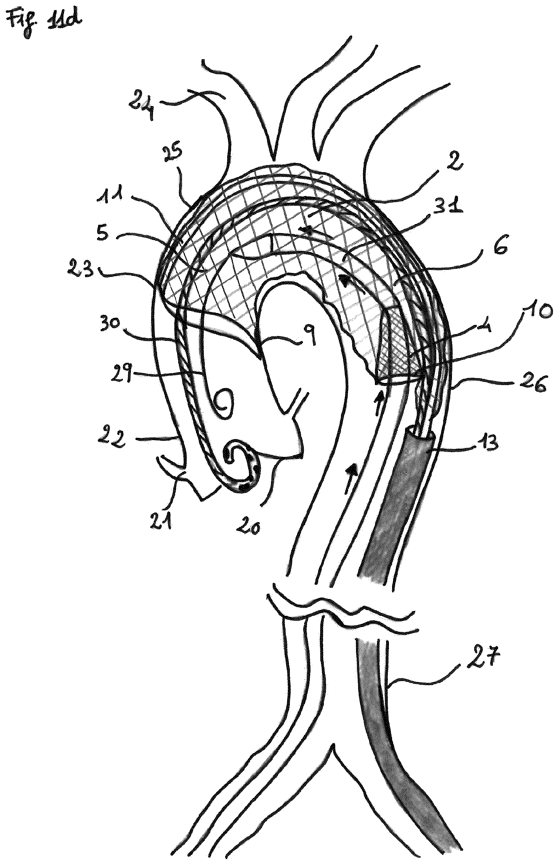

- **Fig. 11d**: **Interaction between the device, pig tail and other working catheters** (31)

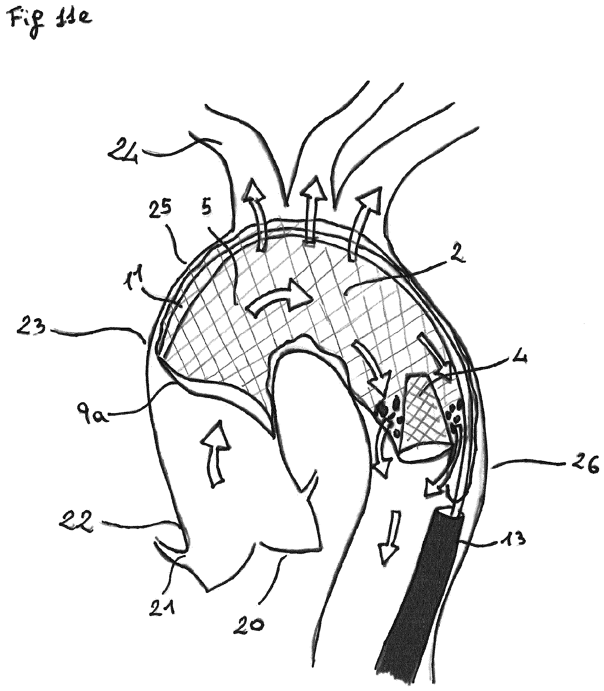

- **Fig. 11e**: **Emboli entrapment and Blood Flow** direction through the device

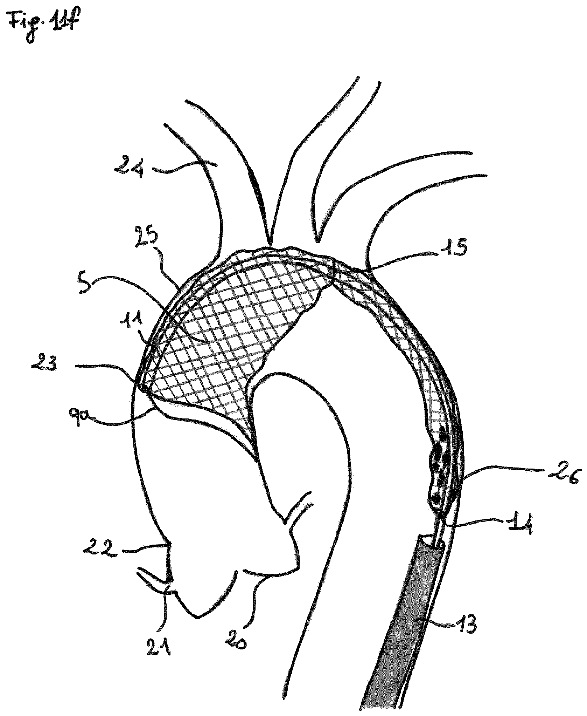

- **Fig. 11f**: **Proximal (14) and distal closure (15) activation**

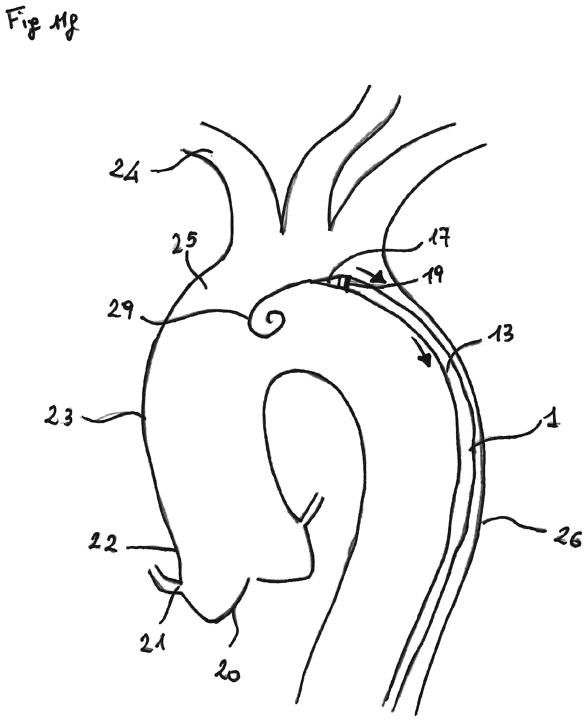

- **Fig. 11g**: **Filter device retrieved** after procedure

### Numerical references used in the figures

### Device related Item

- **1**: transcatheter filter device
- **2**: tubular filter, not limited to a constant section element
- **3**: main body of the tubular filter
- **4**: proximal conical funnel of the tubular filter
- **5**: distal end of the tubular filter
- **6**: proximal end of the tubular filter
- **7**: port of the tubular filter (coincident with the funnel apex)
- **8**: structure assembly
- **9**: distal structure
a. embodiment with one ring
b. embodiment with two rings assembly
- **10**: proximal structure
- **11**: supporting catheter
- **12**: filter-structure-catheter assembly
- **13**: external shaft
- **14**: proximal closure system
a. self-sealing automatic closure mechanism (a-a; a-b: two different embodiments)
b. mechanical closure mechanism (b1 open; b2 closed)
- **15**: distal closure system
- **16**: handle and relevant commands, which can comprise the following elements:
a. command for external shaft (13) movement
b. command for distal structure (9) trim
c. command for funnel (4) movement
d. command for proximal port (7) activation
e. command for distal port (7) activation
f. flushing port for guide wire (29)
g. flushing port for external shaft (13)
- **17**: tip
- **18**: artificial valve
- **19**: radiopaque markers

### Anatomy references

- **20**: Aortic valve
- **21**: Coronary ostia
- **22**: Sinu Tubular Junction
- **23**: Ascending aorta
- **24**: Innominate artery
- **25**: Aortic arch
- **26**: Descending aorta
- **27**: Femoral access

### Working catheters and other accessories

- **28**: Introducer
- **29**: Guidewire
- **30**: Pigtail
- **31**: Working catheters (i.e Delivery system of TAVI, pig tail, annuloplasty device, etc.)

In one embodiment, the antiembolic filter device comprises the following macro elements (Fig. 1): an assembly (12), which includes a tubular filter (2) adapted to retain emboli, whilst allowing blood flow, a structure assembly (8) to sustain the filter and make it couple with a vessel, an external shaft (13) to collapse / track / deploy / retrieve said assembly and a handle (16) to enable with specific commands said operations, together with the optimal sealing with the vessel and the interaction with other devices.

The tubular filter (2) is placed externally to the structure assembly (8), as shown in Fig. 1: this assembly comprises a distal support structures (9), placed upstream respect to the blood flow direction and intended to make a leak-free coupling of the filter with the vessel, a proximal support structure (10), defining the region where the emboli are collected and where other devices pass inside the filter by crossing relevant port (7), and a supporting catheter (11), as shown in Fig. 1c. In specific embodiments, said tubular filter (2), structure assembly (8), esternal shaft (13) and handle (16) are permanently joined.

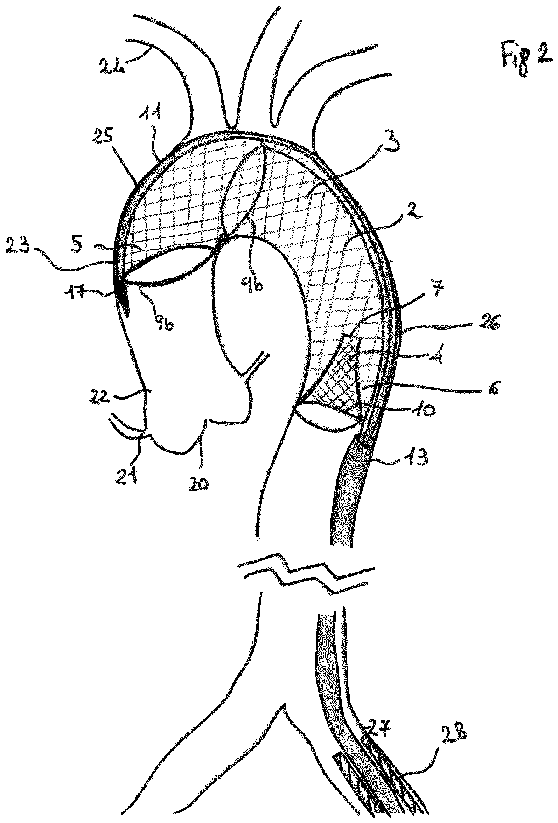

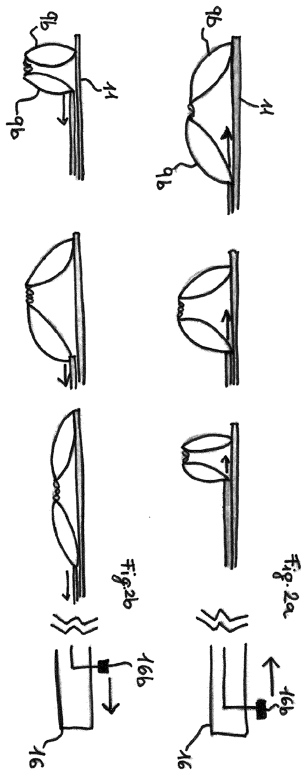

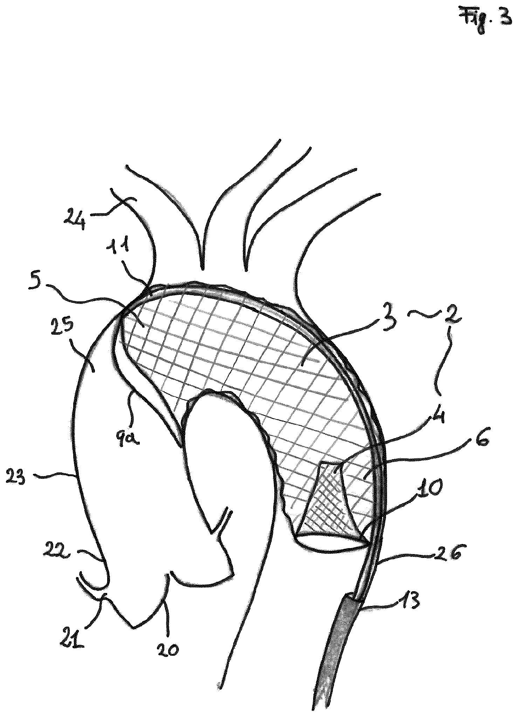

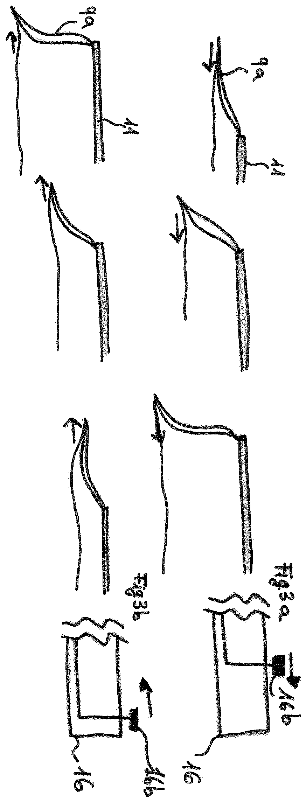

In specific embodiments (Fig. 2, 3), said filter device is adapted to be used as an intra-aortic protection, extending from the ascending aorta (23), upstream with respect to the innominate artery (24), to the descending aorta (26).

Fig. 1a show the main components of said filter device (1) here below described starting from the filter (2) components, then going to the structure (8), shaft (13), handle (16).

The tubular filter (2) is made of a low friction porous and flexible polymeric or composite material, here including polyester or polyamide, with mesh pore preferably lower than 150 microns. It can be coated with either a hydrophilic, low friction or anti-thrombogenic coating or a combination of thereof. Filter material, coating and shape facilitate the navigation of transcatheter devices into its body, both during the insertion and the retrieval, preventing relevant direct contact with the vessel wall, that can make injuries on it. Specific embodiments comprise perforated membranes and fabrics. In one embodiment a woven fabric can be chosen, with warp and weft either made by multifilament or monofilament yarn, with an either constant or variable weaving pattern, thus resulting in a pore comprised amongst the square and the circular geometry and either constant or variable mesh pore and open area along the filter longitudinal and circumferential directions.

The tubular filter (2) is geometrically defined by a distal element and a proximal element, namely a main body (3) and a funnel (4) (Fig. 1a). The main body (3) comprises a distal end (5) and a proximal end (6); said distal end (5) is adapted to be open when the device is in active configuration, hermetically coupled with the vessel and closed before the device retraction; said proximal end (6) that is adapted to be open in the active configuration; said funnel (4) forming an extension of said main body (3), with the funnel base located at said proximal end (6).

Embodiments for the filter main body (3: Figlb) include a cylindrical body, a conical body and combination thereof. Specific embodiments for intra-aortic procedures comprise a three regions main body (referred as 3e in Fig. 1b), with a distal cylindrical part coupling with the aorta (3-1), an intermediate conical part (3-2) having a progressively decreasing diameter and a proximal cylindrical part (3-3), with: said intermediate part shaped to reduce relevant pressure drop for blood circulation and the overall filter encumbrance along the internal side of the aortic arch (25); said proximal cylindrical part geometry intended to allow free forward and back movement of working catheters, even in case of retrieve of partially recollapsed TAVIs in descending aorta. The length of the main body is generally comprised from 10 to 30 cm, in order to be adapted to extend for all the vessel length to be protected from the ascending aorta (23), upstream with respect to the innominate artery (24), to the descending aorta (26).

Embodiments for the filter funnel (4) include movable and fixed funnels, with either symmetric or asymmetric shapes.

Fig.4 shows an embodiment of a movable funnel (4), with funnel in its extreme configurations: a first position (detailed in Fig 4b), in which the funnel apex is proximal with respect to main body (3) proximal open end (6) and a second position (detailed in Fig 4a), in which the funnel apex is positioned within said main body, between said main body (3) distal and proximal ends. In the active configuration, the funnel top is generally positioned inside the main body, thus acting as a sliding conveyor for working catheters that cross it, whilst gathering the emboli in the interspace amongst the main body and the funnel (referred as 4c), this making the interaction between the working catheter and the funnel port intrinsically free from emboli. In this embodiment, the funnel is oriented by acting on the apex, i.e. with a push-pull system commanded by the handle as detailed in Fig. 4b and 4a, this embodiment allowing to move the funnel also whilst a working catheter crosses it.

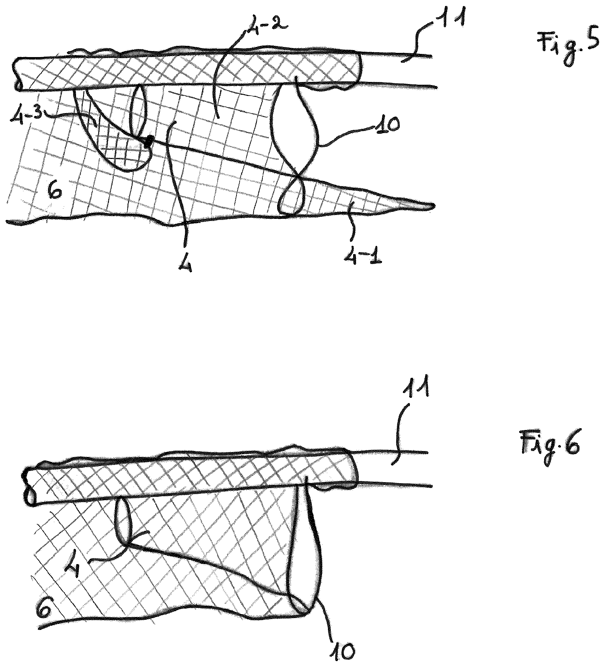

A second embodiment for the funnel (Fig 5) comprises a fixed funnel element with distal apex (4-2), enabling the crossing of working catheters, joined to the following elements: laterally, to a fixed conical element, being the proximal part of the main body (2), with proximal apex (4-1) adapted to collect emboli; to a proximal ring (10, shaped as a "8"), that defines the base of the funnel and of the collecting conical element; to a flap (4-3), with distal end base either connected to the filter main body (2) or to the supporting catheter (11) and proximal end connected at least in a single point to the funnel 4-2, this flap adapted to prevent emboli release downstream, whilst allowing the funnel to be crossed.

In a third embodiment for the funnel (Fig. 6), it comprises an orientation fixed funnel element with the base open at the proximal end in the active configuration, acting as a conveyor; this funnel being joined to at least the following elements: laterally, to a fixed conical element, being the proximal part of the main body (2), with the apex closed at the proximal end; to a radially expandable proximal structure (10), said proximal structure either being manually activated or self-expandable.

The funnel element is generally positioned in a straight portion of the vessel, in order to ensure easy crossing of working catheters at its apex. The funnel is shorter than main body, with a ratio of the funnel to main body length is generally comprised between 1/10 and 1/3, depending on the specific vessel centerline length, shape and vessel diameter. Specific intra-aortic embodiments have a funnel length generally comprised between 2 and 10 cm.

The proximal closure system (14) preventing downstream emboli release, which is positioned at the funnel (4) apex is referred as the filter proximal port (7): it can either consists of a funnel geometry shaped in order to have the apex oriented downstream respect to the blood flow or consists of a folded top or a combination of thereof systems or consists of an actual closure system; an example of closure system is constructed by a lazoo system activated by a wire, either manually (14b) or automatically (14a), thanks to an elastic wire. Fig. 7b shows examples of proximal port applied to a movable funnel systems: in the two pictures at the top, a mechanical closure mechanism is shown in the open (b1) and closed (b2) positions, whilst in the two pictures at the bottom two different self-sealing automatic closure embodiments are shown (a-a; a-b).

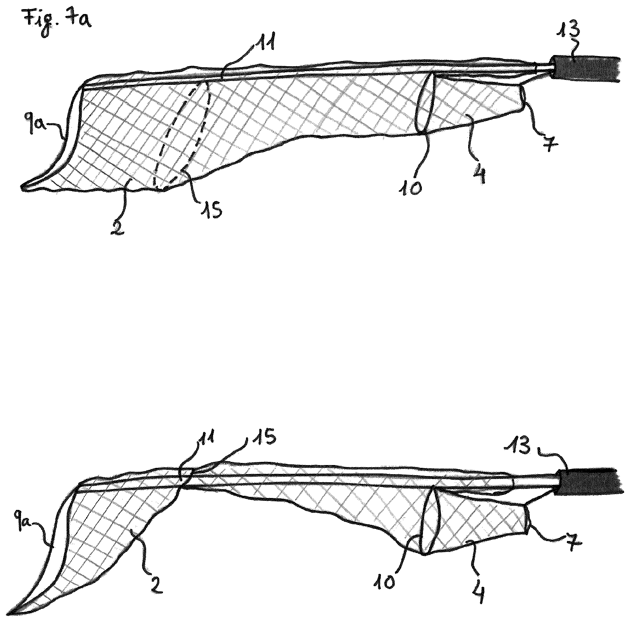

The distal closure system (15), used to prevent upstream dislodgement at the end of the procedure, is activated before recollapsing the device (Fig 7a), being either a lazoo system, manually activated, or an automatic elastic system, which is manually deactivated in the active configuration.

A specific embodiment of the support structure assembly (8) is shown in Fig. 1c; it comprises at least: a supporting catheter (11) extending within the main body, one radially expandable distal structure (9) joined to said main body distal end, one radially expandable proximal structure (10) positioned at said main body proximal end level, said distal structure (9) and said proximal structure (10) being fixed to said supporting catheter (11).

Fig. 2 and Fig. 3 show two intra-aortic embodiments of the filter device (1) in the active configuration, differing on the distal structure (9) element. In both cases, the mechanical stability of the filter device, either in a standalone condition or when crossed by other working catheters, is ensured at least by the coupling of the distal structure (9) with the ascending aorta vessel and by the coupling of the supporting catheter (11) with the aortic arch.

The radial expandable characteristics of the distal structure ensure to cover a broad range of geometry (with ascending aorta diameter usually ranging between 20 and 40 mm) and anatomies with a reduced number of sizes for the filter device without risk of device dislodgment or migration.

In the specific embodiment shown in Fig.2, the distal (9) structure comprises **two rings elements** (9b: here referred as the more proximal and the more distal elements), mutually joined: the more distal ring element is joined to the catheter (11) at its distal end, to the more proximal ring element at its proximal end and to the distal end of the main body (2) along its perimeter; the more proximal element is connected at its proximal end to a specific handle sealing command by a rod, passing inside a lumen of the supporting catheter (11). This structure can be either constructed of a single wire or multiple wires having either a circular, elliptical or rectangular section, or a combination thereof; the joinings amongst the components can be made by crimping, welding, gluing, binding or, in case of wire elements by twisting them, or with a combination of thereof methods.

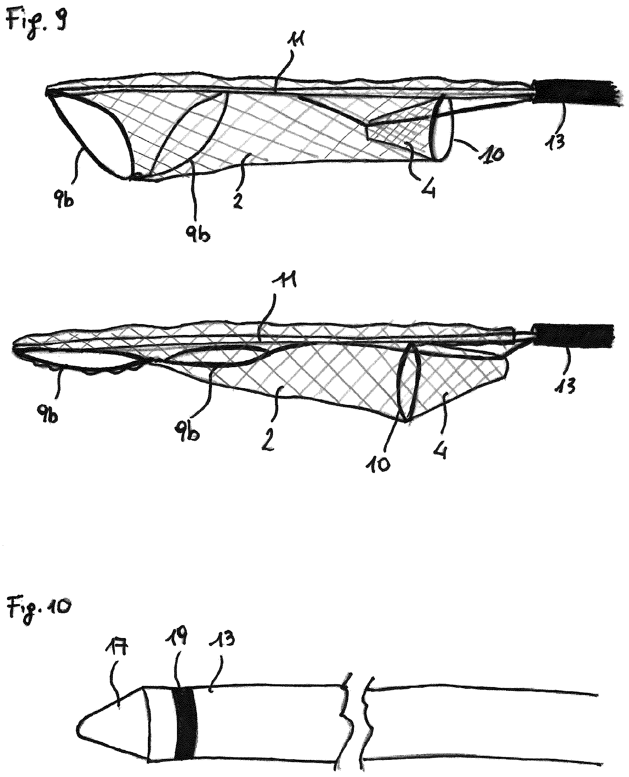

The distal ring element is designed to radially expand conforming to the aorta in the active configuration, thus guaranteeing a leak-free coupling: this is ensured by the high elasticity limit of the material used, preferably but not exclusively being Nitinol, by its geometry, with perimeter larger than the aorta vessel, by relevant axis free orientation, tilted respect to aorta centerline and by relevant deformation mechanisms commanded by the handle. As an example, by actively pushing on the sealing handle command (forward movement on the command 16b: Fig 2a), the proximal ring element pushes onto the distal one, thus partially tilting relevant ring plane and resulting in radial compression onto the aortic wall. In this embodiment the pulling handle command (backward movement on the command 16b: Fig 2b) can be adopted in order to close the filter main body distal end without using specific commands. In a further specific configuration, which is shown in Fig. 9, the interconnection amongst distal ring and funnel commands can act simultaneously on the closure mechanism of the filter distal end and on the movement of the funnel apex, thus simplifying the handle mechanism and the operations to be carried out prior to the device retrieve.

In the embodiment shown in Fig.3, the distal (9) structure comprises **one ring element** (9a), with proximal end joined to the catheter (11), perimeter joined at the distal end of the main body (2), distal end joined to a single or multiple wire passing inside the filter and connected to a specific handle sealing command. In this case the radial expansion of the ring (Fig 3a), for which apply considerations similar to the distal ring element referred in Fig. 2a, is ensured by a pulling system rather than a pushing system command.

A specific embodiment of the proximal structure (10) is shown in Fig. 4, with structure shaped as a ring and connected to the supporting catheter. The ring defines the base of the funnel (4), allowing to orient it either in the proximal and distal directions, by tilting its apex by specific commands connected to the handle. In the embodiment shown, the ring doesn't couple with the descending aorta. In other embodiments the proximal structure (10) can be shaped similarly respect to the distal structure (9), thus allowing to radially couple with the aorta vessel.

Specific embodiments can be constructed wherein structures intermediate respect to the distal (9) and proximal (10) can be connected to the supporting catheter (11), in order to increase the device stability and contribute to fully expand the tubular filter main body.

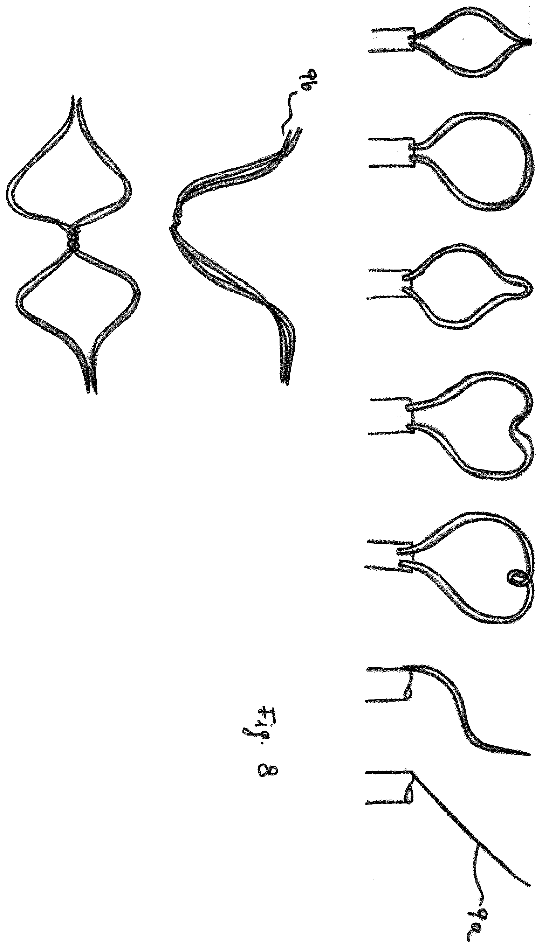

For both the distal and proximal, and where applicable intermediate, structures, the overall geometry can be elliptical in plan view, but also differently shaped as shown in Fig. 8; similarly, lateral view can show a planar structure but also a "S" shaped lateral profile to enhance leak-free conforming to the aortic arch, as shown in Fig. 8.

The supporting catheter (11), which is joined to the distal (9) and proximal (10) structures and to the tubular filter (2), adapts in the active configuration at the extrados of the aortic arch and sustains all the loads arising from the procedure (see Fig. 11): at this purpose it can be made by a flexible polymeric or a composite material, here including a metal braided polymer, selected as the best compromise amongst high elongation / compression / torsion stiffness and fairly high flexibility. The supporting catheter (11) profile is adapted to house inside specific lumens, the commands to crimp / deploy the filter (2), to act on the distal (9) and proximal (10) structures and on the distal and proximal filter closure system, where applicable, and to house other accessories / working catheters, here including guide-wire, pig-tail and balloon catheters, here contributing to simplify the overall procedure.

The external shaft (13: see details in Fig. 10 and 11) is adapted to guide the collapsed filter assembly (12) in position and to allow the deployment/recapture of the device, by sliding backward and forward respect to the multilumen catheter. The external shaft (13) is made of a flexible polymeric or composite material and preferably a metal braided polymer, i.e with reduced tensile and compression elongation and with adequate flexural compliance to ensure optimal pushability when tracking the filter device along the aortic arch, thus allowing to adapt to the extrados curvature of aorta without forcing onto it and minimizing snacking whilst interacting with the supporting catheter to crimp / deploy the filter.

A tip (17) can be included in any of the said structures (9, 11) or external shaft (13) or other structures to allow adequate priming, easy crossing of the introducer and smooth navigation into the aorta (Fig.2 and Fig.10).

Radiopaque markers (19: see details in Fig.10, Fig.11b) can be joined to specific locations (11, 13, 9, 10 or other structures) in order to facilitate, via adequate imaging, the positioning of the device (1) and of other working catheters intended to cross it.

The handle (16: Fig.1a) allows specific commands including, where applicable, the sliding between an external shaft (13) and the supporting catheter (11), thus allowing filter crimping or deployment (16a), the activation of the distal closure mechanism (16e), the activation of proximal (14: 16d) and distal (15: 16e) closure mechanisms, the tensioning of the distal support structure (9: 16b), the funnel movement (16c), the flushing of the ports for guide wire (16f) and external shaft (16g), the direct loading of other devices or accessories, not limited to a guide wire and / or a pig tail catheter and the enabling / disabling of an artificial valve (18), where applicable. The handle structure is made preferably but not exclusively by polymeric material; it houses all the commands, either made by rods or wires, and the proximal terminations of the supporting catheters (11) and of the external shaft (13), either directly or with the interposition of metal tubes. The handle can adopt either linear / rotatory mechanism to allow said movements and block systems, where applicable, to fix it in a determined position.

Here below a transcatheter procedure adopting the antiembolic filter device (1) is detailed, with specific features referring to an intra-aortic procedure, here comprising a TAVI, which allow cerebral and systemic emboli protection.

In this example, the antiembolic filter device (1) access is made from the femoral artery opposite (secondary) to that one (main) accessed by the working catheter (31) used for the prosthesis or the device to treat the aortic valve (Fig.11d). Here below relevant procedure details:
a) An introducer (28) is inserted inside a femoral access (27).
b) A guide wire (29) is inserted inside the introducer (28) and navigated up to the aortic arch (Fig. 11a). As an option a pigtail is inserted and navigated up to the ascending aorta to allow a fluoroscopic imaging reference prior to the filter device insertion.
c) The filter device is collapsed, primed and debubbled into the external shaft catheter (13) before to introduce it into the arterial vessel (Fig. 11b).
d) The device is tracked along the vessel and positioned, with the aid of radiopaque markers and adequate imaging technique, the upstream respect to the innominate artery (24); the device is deployed and coupled with ascending aorta, by retracting the external shaft catheter (13) (Fig. 11c).
e) When the device is deployed, the distal end of the Filter (5) is fitting the aortic wall in order to convey all blood and possible debris into its funnel (Fig.11e) thanks to the support structure (9), which circumferentially push the distal filter surface (5) against the aortic wall.
f) It is now possible to introduce the other working catheters inside the filter by crossing the funnel (4), whilst the funnel (4) and port (7) configuration prevents forward debris dislodgment. Fig.11d shows the interaction amongst filter device and other working catheters normally used in a TAVI procedure.
g) At the end of the procedure the distal end of the filter (5), which in the expanded configuration remains open, can be closed, before the device recollapsing, in order to prevent any upstream debris dislodgment of the emboli collected at the proximal end of the device (Fig.11f).
h) The device is completely re-collapsed by pushing distally the external shaft catheter (13), as shown in Fig. 11g. In this way, the device structures gradually collapse until reaching the distal end of the device safely keeping inside it all captured clots or calcium debris.
i) Finally, the overall device is retrieved.

Specific procedures can require partial closure, repositioning and re-deployment at different levels (e.g. from sinotubular junction to descending aorta) and, eventually, different supporting catheter positioning. Moreover, procedures different respect to intra-aortic ones can require different geometrical arrangements of the above-mentioned concepts, therefore this filter device can be applied in principle in any arterial or venous system requiring an antiembolic protection.

This method allows to deploy the filter device prior to the other working catheters (31: see Fig.11d) and retrieve it after all the other working catheters, thus enabling to:
a) collect and retain emboli released during transcatheter procedures, with working devices eventually moving along the filter;
b) track working catheters inside the filter without direct contact to the vessel wall;
c) have the proximal port (14) of the filter generally closed during the whole procedure and the distal port (15) closed before the filter device is recollapsed and retrieved, thus preventing any downstream and upstream emboli release (Fig.11f).

## Claims

1. An intra-vessel transcatheter filter device (1) designed to capture and remove emboli, thus preventing distal embolization, comprising:
• a tubular filter (2) comprising a flexible porous material and defined by a distal element and a proximal element, namely a main body (3) and a funnel (4);
i. said main body (3) having a length adapted to extend within a suitable vessel zone; said main body (3) comprising:
a) a distal end (5) that is adapted to be radially coupled with said zone and hermetically sealed to it when the device is in an active configuration, said distal end (5) being provided of selectively actionable closure means so that said distal end is designed to be open when the device is in an active configuration and closed before the device retraction,
b) a proximal end (6);
ii. said funnel (4) forming an extension of said main body (3), with the funnel base located at said proximal end (6).
• a support structure assembly (8) comprising:
i. a supporting catheter (11) extending within said main body (3),
ii. one radially expandable distal structure (9) fixed to said distal end (5),
iii. one radially expandable proximal structure (10) positioned in correspondence of said proximal end (6),
said distal structure (9) and said proximal structure (10) being fixed at least in an end to said supporting catheter (11).

2. Device according to claim 1 wherein the funnel (4), having substantially the same cross-section area of the main body (2), is movable with respect to the main body (3), between a first position in which the funnel top is positioned outside said main body (3) and a second position in which the funnel top is positioned within said main body (3), between said distal end (5) and said proximal end (6).

3. Device according to claim 1 wherein the funnel (4) is fixed with respect to the main body (3), with the funnel top being permanently positioned within said main body (3), between said distal end (5) and said proximal end (6).

4. Device according to anyone of the previous claims wherein the porosity of said flexible porous mesh material of the filter (2) is lower than 150 micron, and preferably between 40 and 70 microns.

5. Device according to anyone of the previous claims wherein said distal (5) and proximal (6) ends are both provided of selectively actionable closure mechanisms.

6. Device according to anyone of the previous claims wherein the filter is made of a low friction and flexible polymeric or composite material, preferably but not exclusively a fabric.

7. Device according to anyone of the previous claims wherein the filter is coated with either a hydrophilic, low friction or anti-thrombogenic coating or a combination of thereof.

8. Device according to anyone of the previous claims wherein the distal (9) and proximal (10) structures have a ring shape.

9. Device according to claim 8 wherein at least one of the said structures (9, 10) comprises two rings that are mutually joined.

10. Device according to anyone of the previous claims wherein the distal structure (9) is part of said selectively actionable closure mechanisms.

11. Device according to anyone of the previous claims wherein the funnel (4) apex is provided with a catheter access port, so that the funnel (4) is designed to act as a conveyor for working catheters that cross it.

12. Device according to anyone of the previous claims wherein said catheter (11) is adapted to track inside relevant lumen other working catheters or instruments, whilst allowing emboli retaining.

13. Device according to anyone of the previous claims, having a geometry adapted to intra-vessel procedures and length of the filter main body adapted to extend within a suitable vessel zone, e.g. for intra-aortic procedures generally comprised between 10 cm and 30 cm in order to be adapted to extend from the ascending aorta (23) to the descending aorta (26).

14. Device according to anyone of the previous claims comprising an artificial valve (18).

15. Method for emboli protection during intra-vessel procedures, adopting a transcatheter device as detailed in claim 1; the method comprising the following steps:
• Inserting via a suitable access (27) an antiembolic tubular filter (2), comprising a supporting catheter (11) and an expandable support structure (9, 10) collapsed into an external shaft (13) and tracked along a guidewire (29);
• Positioning, with the aid of radiopaque markers and adequate imaging technique, the distal end of the filter at the intended position, i.e. for an intra-aortic procedure generally but not exclusively in the ascending aorta, upstream to the innominate artery;
• Proximally retrieving the external shaft in order to fully deploy the filter from the distal to the proximal end, thus generally giving to the filter a configuration with two generally open ends - the distal (5) sealing to the aorta wall, the proximal (6) acting as the base of a funnel (4);
• Enabling specific working instruments to enter inside the filter by crossing the funnel (4) apex, with funnel and main body configured to prevent emboli downstream release.
• Enabling the proximal closure system for the whole procedure and the distal closure system before filter retieve.
